(19) **Europäisches Patentamt / European Patent Office / Office européen des brevets**

(11) **EP 4 364 813 A1**

(12) **EUROPEAN PATENT APPLICATION**
published in accordance with Art. 153(4) EPC

(43) Date of publication:
**08.05.2024 Bulletin 2024/19**

(21) Application number: **22833245.8**

(22) Date of filing: **29.06.2022**

(51) International Patent Classification (IPC):
*A61Q 1/00* (2006.01)    *A61Q 19/00* (2006.01)
*A61K 8/11* (2006.01)    *A61K 8/25* (2006.01)
*A61K 8/73* (2006.01)    *B01J 13/14* (2006.01)

(52) Cooperative Patent Classification (CPC):
**A61K 8/11; A61K 8/25; A61K 8/73; A61Q 1/00;
A61Q 19/00; B01J 13/14**

(86) International application number:
**PCT/JP2022/026083**

(87) International publication number:
**WO 2023/277099 (05.01.2023 Gazette 2023/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.06.2021  JP 2021109562**

(71) Applicant: **Kao Corporation
Tokyo 103-8210 (JP)**

(72) Inventors:
• **KOGA, Yoshito
Wakayama-shi, Wakayama 640-8580 (JP)**
• **FUKUZUMI, Keita
Wakayama-shi, Wakayama 640-8580 (JP)**

(74) Representative: **Hoffmann Eitle
Patent- und Rechtsanwälte PartmbB
Arabellastraße 30
81925 München (DE)**

(54) **AQUEOUS MICROCAPSULE DISPERSION**

(57)    The present invention relates to an aqueous microcapsule dispersion liquid containing a component (A) and a component (B) below: component (A): microcapsules having a shell containing an inorganic material as a constitutional component, and a core containing one or more kind of an organic compound, inside the shell, and component (B): a polysaccharide including a cationically modified branched chain structure.

**Description**

Field of Invention

The present invention relates to an aqueous microcapsule dispersion liquid.

Background of the Invention

In a broad business field including cosmetics, medicines, general household products, and printing, various microcapsules encapsulating a fragrance material or a physiologically active substance therein have been developed and utilized. The methods having been used for producing the microcapsules include a chemical method, such as a suspension polymerization method, a mini-emulsion polymerization method, an emulsion polymerization method, a precipitation polymerization method, a dispersion polymerization method, an interfacial polycondensation method, and a submersible curing method; a physicochemical method, such as a submersible drying method, a phase-transfer emulsification method, and a coacervation method; and a mechanical method, such as a spray-drying method and a heteroaggregation method. Many of the production methods of microcapsules provide an aqueous dispersion liquid of microcapsules dispersed in an aqueous medium, and from the industrial standpoint, it is desired to use the resulting aqueous dispersion liquid of microcapsules directly without performing an isolation operation, such as filtration or drying.

Under the circumstances, various investigations have been made for aqueous dispersion liquids of microcapsules.

EP 2,862,597 A (PTL 1) describes a stable silica capsule composition having fluidity constituted by a silica capsule suspension liquid and an adjuvant, in which the adjuvant used is a cationic polymer, such as a vinylpyrrolidone-dimethylaminoethyl methacrylate copolymer.

WO 2018/053356 A (PTL 2) intends to enhance the dispersion stability of a microcapsule composition with a viscosity modifier, and describes a microcapsule composition containing microcapsules dispersed in a water phase, and a viscosity modifier, in which the viscosity modifier described is an acrylate copolymer, a cationic acrylamide copolymer, or a polysaccharide.

JP 2012-501849 A (PTL 3) intends to enhance the stability of an aqueous suspension liquid of microcapsules having a silicate shell, and describes an aqueous suspension liquid of microcapsules having a silicate shell using an organic functional silane as a colloidal silicate sequestering agent.

Summary of the Invention

The present invention relates to an aqueous microcapsule dispersion liquid containing a component (A) and a component (B) below.

    Component (A): microcapsules having a shell containing an inorganic material as a constitutional component, and a core containing one or more kind of an organic compound, inside the shell
    Component (B): a polysaccharide including a cationically modified branched chain structure

Detailed Description of the Invention

An aqueous dispersion liquid of microcapsules has a problem of decreasing fluidity due to the difference in specific gravity between the microcapsules and the aqueous medium, the viscosity of the aqueous medium, and the particle diameter and the shape of the microcapsules. For example, in the case where the specific gravity of the microcapsules is relatively smaller than the aqueous medium, creaming occurs with the lapse of time to lose fluidity, and in the case where the specific gravity of the microcapsules is relatively larger, sedimentation occurs with the lapse of time to lose fluidity.

An aqueous dispersion liquid of microcapsules also has a problem of losing fluidity of the aqueous dispersion liquid due to the occurrence of gelation or formation of aggregates through physical or chemical change of the surface of the shell constituting the microcapsules or the component contained in the aqueous medium.

However, the techniques of PTLs 1 to 3 are insufficient in dispersion stability of microcapsules in the aqueous dispersion liquid. Microcapsules have been applied to a wide range of business fields, but cannot be sufficiently satisfactory from the standpoint of the handleability thereof in the form of an aqueous dispersion liquid. Under the circumstances, there is a demand of an aqueous dispersion liquid of microcapsules that is suppressed in formation of aggregates and suppressed in decrease in fluidity even in long-term storage, and is excellent in dispersion stability.

Furthermore, from the standpoint of controlling the sustained-release capability of an organic compound encapsulated in microcapsules, there is a demand of an aqueous dispersion liquid of microcapsules that has a favorable particle diameter of the microcapsules, has a small variation of the particle diameter of the microcapsules, and is excellent in uniformity in particle diameter, even after long-term storage of the aqueous dispersion liquid of microcapsules.

The present invention relates to an aqueous dispersion liquid of microcapsules that is excellent in dispersion stability, has a favorable particle diameter of the microcapsules, has a small variation of the particle diameter thereof, and is excellent in uniformity in particle diameter thereof, even after long-term storage.

The present inventors have focused the fact that in an aqueous dispersion liquid of microcapsules having a shell containing an inorganic material as a constitutional component and a core containing one or more kind of an organic compound, inside the shell, a polysaccharide including a cationically modified branched chain structure contained therein enhances the dispersion stability of the microcapsules in the aqueous dispersion liquid, achieves a favorable particle diameter of the microcapsules, has a small variation of particle diameter thereof, and enhances the uniformity in particle diameter thereof, even after long-term storage, and thus have found that an aqueous dispersion liquid of microcapsules can be provided.

Specifically, the present invention relates to an aqueous microcapsule dispersion liquid containing a component (A) and a component (B) below:

component (A): microcapsules having a shell containing an inorganic material as a constitutional component, and a core containing one or more kind of an organic compound, inside the shell, and
component (B): a polysaccharide including a cationically modified branched chain structure.

The present invention can provide an aqueous dispersion liquid of microcapsules that is excellent in dispersion stability, has a favorable particle diameter of the microcapsules, has a small variation of the particle diameter thereof, and is excellent in uniformity in particle diameter thereof, even after long-term storage.

[Aqueous Microcapsule Dispersion Liquid]

The aqueous microcapsule dispersion liquid (which may be hereinafter referred simply to as an "aqueous dispersion liquid") of the present invention contains a component (A) and a component (B) below.

Component (A): microcapsules having a shell containing an inorganic material as a constitutional component, and a core containing one or more kind of an organic compound, inside the shell
Component (B): a polysaccharide including a cationically modified branched chain structure

In the description herein, the uniformity of the particle diameter of the microcapsules with a small variation of the particle diameter thereof after long-term storage may be hereinafter referred to as "particle diameter uniformity".

In the present invention, the "polysaccharide including a cationically modified branched chain structure" means a polysaccharide in which at least one constitutional component among the constitutional components of the cationically modified polysaccharide has a cationically modified branched chain structure.

In other words, the "polysaccharide including a cationically modified branched chain structure" in the present invention is a concept that encompasses a polysaccharide having a cationically modified branched chain structure alone, and a mixture containing a polysaccharide having a cationically modified branched chain structure and a polysaccharide having a cationically modified linear chain structure.

In the present invention, the "polysaccharide including a cationically modified branched chain structure" means a modified polysaccharide having a polysaccharide skeleton having three or more of one or more kind of a monosaccharide connected to each other, to which a cationic group is introduced, in which the structure of the modified polysaccharide is a branched chain structure having at least one monosaccharide molecule that has three or more bonding sites to the adjacent monosaccharide molecule.

On the other hand, the "polysaccharide including a cationically modified linear chain structure" means a modified polysaccharide having a polysaccharide skeleton having three or more of one or more kind of a monosaccharide connected to each other, to which a cationic group is introduced, in which the structure of the modified polysaccharide is a linear chain structure having two bonding sites to the adjacent monosaccharide molecule in one monosaccharide molecule.

The cationic group herein means a cation group or a group capable of becoming a cation group through ionization, and means, for example, a quaternary ammonium group or a group capable of becoming a cation through addition of proton.

In the description herein, the "polysaccharide including a cationically modified branched chain structure" may also be referred simply to as a "cationized branched polysaccharide".

The aqueous microcapsule dispersion liquid of the present invention contains the microcapsules as the component (A) dispersed in an aqueous medium.

In the description herein, the "aqueous medium" is a liquid containing at least water, and preferably contains water occupying the largest proportion in the medium. Examples of the component other than water capable of being contained in the aqueous medium include an aliphatic alcohol having 1 or more and 4 or less carbon atoms; a ketone compound having 3 or more and 8 or less carbon atoms; an ether compound, such as ethyl ether and tetrahydrofuran; and an ester compound, such as methyl acetate.

The content of water in the aqueous medium is preferably 80% by mass or more, and more preferably 90% by mass or more, is also 100% by mass or less, and further preferably 100% by mass, from the standpoint of enhancing the dispersion stability of the microcapsules, and the standpoint of achieving the favorable particle diameter of the microcapsules and enhancing the particle diameter uniformity. Preferred examples of water used include ion exchanged water, deionized water, and distilled water.

In the present invention, the expression "containing the component (A) and the component (B)" also means "formed by blending the component (A) and the component (B)".

In the present invention, the microcapsules as the component (A) in the aqueous dispersion liquid may form flocculate.

The "flocculate" in the description herein means a state of the microcapsules dispersed in the aqueous dispersion liquid that form a mass in the aqueous dispersion through still standing or the like.

In the present invention, even after the microcapsules dispersed in the aqueous dispersion liquid form flocculate, the microcapsules can return to the original dispersed state in the aqueous dispersion liquid through agitation or shaking. From this standpoint, the "flocculate" in the description herein is a reversible phenomenon. On the other hand, the "aggregation" in the description herein means a state in which the microcapsules dispersed in the aqueous dispersion liquid form an aggregate in the aqueous dispersion through still standing or the like. The aggregate cannot return to the original dispersed state in the aqueous dispersion liquid even through agitation or shaking is performed. The aggregate may be redispersed in some cases through cracking, i.e., breaking the aggregation of the aggregate by applying a physical force to the aggregate, and the state that cannot be redispersed by agitation or shaking is designated as the "aggregation". From this standpoint, the "aggregation" in the description herein is an irreversible phenomenon through agitation or shaking as a dispersing means.

In the description herein, the "agitation" and the "shaking" in redispersing the microcapsules each mean an operation of applying an external physical force to the aqueous dispersion liquid in such a manner that the microcapsules are prevented from being damaged thereby as much as possible, so as to allow the system to return to the uniform dispersed state.

The reason why the effects of the present invention can be attained, while not entirely clear, will be considered as follows.

In the present invention, the polysaccharide including a cationically modified branched chain structure (i.e., the cationized branched polysaccharide) has multiple hydroxy groups and cationic groups. Accordingly, it is considered that in the aqueous dispersion liquid, the hydroxy groups of the cationized branched polysaccharide are adsorbed on the surface of the microcapsules, and simultaneously the polymer chains of the cationized branched polysaccharide are expanded in the aqueous medium through electrostatic repulsion between the cationic groups of the cationized branched polysaccharide and the positive charge on the surface of the microcapsules, resulting in the steric repulsive force and the electrostatic repulsive force among the microcapsules, which synergistically act to suppress the formation of aggregates of the microcapsules. In the present invention, furthermore, it is considered that the cationized branched polysaccharide has a branched chain structure, and thereby prevents the occurrence of so-called crosslinking aggregation, i.e., the phenomenon in which one polymer chain is adsorbed simultaneously to the surfaces of the multiple microcapsules to aggregate the microcapsules, and consequently the steric repulsive force and the electrostatic repulsive force among the microcapsules described above more effectively act to suppress the formation of aggregates of the microcapsules, resulting in the enhancement of the dispersion stability of the microcapsules, the achievement of the favorable particle diameter of the microcapsules, and the enhancement of the particle diameter uniformity.

<Component (A)>

The aqueous microcapsule dispersion liquid of the present invention contains microcapsules having a shell containing an inorganic material as a constitutional component, and a core containing one or more kind of an organic compound, inside the shell, as the component (A).

The inorganic material constituting the shell of the component (A) is preferably a metal oxide containing a metal element or a semimetal element, and more preferably an inorganic polymer formed through sol-gel reaction using a metal alkoxide ($M(OR)_x$) as a shell precursor, in which M represents a metal or a semimetal element, and R represents a hydrocarbon group. Examples of the metal or semimetal element constituting the metal alkoxide include silicon, aluminum, titanium, zirconium, and zinc.

The inorganic material is further preferably an inorganic polymer formed through sol-gel reaction using a metal alkoxide of one or more kind selected from the group consisting of silicon, aluminum, and titanium as a shell precursor, and still further preferably silica formed through sol-gel reaction using an alkoxysilane as a shell precursor, from the standpoint of enhancing the dispersion stability of the microcapsules, and the standpoint of achieving the favorable particle diameter of the microcapsules, and enhancing the particle diameter uniformity, and the standpoint of reducing the environmental burden in releasing to the external environment. Accordingly, the component (A) is preferably microcapsules having a shell containing silica as a constitutional component, and a core containing one or more kind of an organic compound, inside the shell (i.e., silica microcapsules) (which may be hereinafter referred to as "silica capsules").

The alkoxysilane is preferably a tetraalkoxysilane from the standpoint of enhancing the dispersion stability of the microcapsules, and the standpoint of achieving the favorable particle diameter of the microcapsules, and enhancing the particle diameter uniformity.

The tetraalkoxysilane is preferably a tetraalkoxysilane having an alkoxy group having 1 or more and 4 or less carbon atoms, more preferably one or more kind selected from the group consisting of tetramethoxysilane, tetraethoxysilane, and tetraisopropoxysilane, further preferably one or more kind selected from the group consisting of tetramethoxysilane and tetraethoxysilane, and still further preferably tetraethoxysilane, from the same standpoint as above.

The organic compound contained in the core of the component (A) is preferably one or more kind selected from the group consisting of a fragrance material; a fragrance precursor; an oil; an antioxidant; an antibacterial agent; a fertilizer; a surface modifier for fibers, skin, hair, and the like; a cooling agent; a dye; a colorant; a silicone; a solvent; and an oil soluble polymer, more preferably one or more kind selected from the group consisting of a fragrance material, a fragrance precursor, an oil, an antioxidant, an antibacterial agent, a fertilizer, a surface modifier, and a solvent, further preferably one or more kind selected from the group consisting of a fragrance material, a fragrance precursor, an oil, an antioxidant, and a solvent, still further preferably one or more kind selected from the group consisting of a fragrance material, a fragrance precursor, and an oil, and still more further preferably one or more kind selected from the group consisting of a fragrance material and a fragrance precursor.

The organic compounds may be appropriately combined depending on the application of the microcapsules.

Examples of the fragrance precursor include a compound releasing a fragrance component through reaction with water, and a compound releasing a fragrance component through reaction with light.

Examples of the compound releasing a fragrance component through reaction with water include a silicate ester compound having an alkoxy component derived from a fragrance alcohol, a fatty acid ester compound having an alkoxy component derived from a fragrance alcohol, an acetal compound or a hemiacetal compound obtained through reaction of a carbonyl component derived from a fragrance aldehyde or a fragrance ketone and an alcohol compound, a Schiff base compound obtained through reaction of a carbonyl component derived from a fragrance aldehyde or a fragrance ketone and a primary amine compound, and a hemiaminal compound or a hydrazone compound obtained through reaction of a carbonyl component derived from a fragrance aldehyde or a fragrance ketone and a hydrazine compound.

Examples of the compound releasing a fragrance component through reaction with light include a 2-nitrobenzyl ether compound having an alkoxy component derived from a fragrance alcohol, an $\alpha$-keto ester compound having a carbonyl component derived from a fragrance aldehyde or a fragrance ketone, and a coumarate ester compound having an alkoxy component derived from a fragrance alcohol. These fragrance precursors each may be, for example, a polymer, such as a reaction product of a part of a carboxy group of a polyacrylic acid and a fragrance alcohol.

The organic compound preferably has appropriate hydrophobicity from the standpoint of the retainability of the organic compound. As an index showing the hydrophilicity or hydrophobicity of the organic compound, a cLogP value that is the calculated value of common logarithm of the distribution coefficient P between n-octanol and water (n-octanol/water) "logP" may be used. The cLogP value herein is the "LogP (cLogP)" calculated by the method described in A. Leo Comprehensive Medicinal Chemistry, Vol. 4 C. Hansch, P.G. Sammens, J.B. Taylor and C.A. Ramsden, Eds., P. 295, Pergamon Press, 1990, and is a cLogP value that is calculated with a program, CLOGP v. 4.01.

In the case where the organic compound is constituted by multiple kinds of constitutional components, the cLogP value of the organic compound can be obtained in such a manner that the cLogP values of the constitutional components are multiplied by the volume proportions of the constitutional components respectively, and the resulting values are summated.

The cLogP value of the organic compound is preferably 1 or more, more preferably 2 or more, further preferably 3 or more, and still further preferably 4 or more, and is also preferably 30 or less, more preferably 20 or less, and further preferably 10 or less.

The shell of the silica capsules encapsulates the core, contains silica as a constitutional component, and preferably has an average thickness of 5 nm or more and 20 nm or less.

The shell of the silica capsules is preferably a multilayer shell including an inner shell containing silica, which is a hydrolytic polycondensation product of an alkoxysilane, as a constitutional component, and an outer shell containing silica, which is a hydrolytic polycondensation product of an alkoxysilane, as a constitutional component, outside the inner shell, from the standpoint of enhancing the dispersion stability of the microcapsules, and the standpoint of the retainability of the organic compound. Specific examples of the silica capsules include the silica capsules described in JP 2015-128762 A.

In the case where the shell of the silica capsules is the multilayer shell including the inner shell and the outer shell, the inner shell encapsulates the core, contains silica as a constitutional component, and preferably has an average thickness of 5 nm or more and 20 nm or less, and the outer shell encapsulates the inner shell, contains silica as a constitutional component, and preferably has an average thickness of 10 nm or more and 100 nm or less.

The average thickness of the shell of the silica capsules and the average thicknesses of the inner shell and the outer shell of the silica capsules can be measured with a transmission electron microscope (TEM). Specifically, under observation with a transmission electron microscope, the thicknesses of the shell or the inner shell and the outer shell are actually measured on the micrograph. This operation is repeated five times while changing the view fields. The distribution of the average thickness of the shell or the inner shell and the outer shell is obtained from the resulting data. The magnification of the transmission electron microscope is 10,000 or more and 100,000 or less as a rough standard, and may be appropriately regulated depending on the size of the silica capsules. Examples of the transmission electron microscope (TEM) used herein include "JEM-2100", trade name, available from JEOL, Ltd.

The component (A) used may be appropriately synthesized.

In the case where the component (A) is silica capsules, preferred examples of the silica capsules include those produced by a method including the following step I.

Step I: A step of subjecting an emulsion liquid obtained by emulsifying a water phase component containing a cationic surfactant and an oil phase component containing the organic compound and a tetraalkoxysilane, to sol-gel reaction under an acidic condition, so as to form silica capsules having a core and a shell containing silica as a constitutional component, resulting in an aqueous dispersion containing the silica capsules

Examples of the cationic surfactant used in the step I include an alkylamine salt and an alkyl quaternary ammonium salt. The number of carbon atoms of the alkylamine salt and the alkyl quaternary ammonium salt is preferably 10 or more and 22 or less.

The content of the cationic surfactant in the water phase component in the step I is preferably 0.05% by mass or more and 10% by mass or less, from the standpoint of the dispersion stability of the emulsion droplets.

The amount of the tetraalkoxysilane used in the step I is preferably 10 parts by mass or more from the standpoint of accelerating the sol-gel reaction and forming a sufficiently dense shell, and is also preferably 60 parts by mass or less from the standpoint of suppressing the tetraalkoxysilane from remaining in the organic compound, all per 100 parts by mass of the organic compound used in the step I.

The amount of the oil phase component in the total amount of the emulsion liquid used in the step I is preferably 5% by mass or more and 50% by mass or less from the standpoint of the production efficiency.

The agitation means used in the preparation of the emulsion liquid is not particularly limited, and a homogenizer, a high pressure disperser, an ultrasonic disperser, and the like, having a large shearing force may be used.

The temperature in mixing and emulsifying the water phase component and the oil phase component is preferably 5°C or more and 50°C or less from the standpoint of the production stability.

The rotation number and the like of the agitation means and the period of time of mixing and emulsifying the water phase component and the oil phase component may be appropriately regulated to provide a median diameter $D_{50}$ of emulsion droplets of the emulsion liquid within the range described later.

The median diameter $D_{50}$ of the emulsion droplets of the emulsion liquid in the step I is preferably 0.1 $\mu$m or more from the standpoint of reducing the specific surface area of the silica capsules facing the external environment, and enhancing the retainability of the organic compound, and is also preferably 50 $\mu$m or less from the standpoint of reducing the particle diameter of the silica capsules and the standpoint of the mechanical strength of the silica capsules.

The median diameter $D_{50}$ of the emulsion droplets can be measured according to the method shown in the examples.

The initial pH of the sol-gel reaction in the step I is preferably 3.0 or more from the standpoint of retaining the balance between the hydrolytic decomposition reaction and the condensation reaction of the tetraalkoxysilane and the standpoint of suppressing the sol having high hydrophilicity and accelerating the progress of the encapsulation reaction, and is also preferably 4.5 or less from the standpoint of suppressing the simultaneous occurrence of the formation of the silica shell and the aggregation of the emulsion droplets, and providing the silica capsules having a dense shell.

The pH of the emulsion liquid may be regulated by using a pH modifier depending on the strength of acidity or alkalinity of the oil phase component containing the organic compound from the standpoint of regulating to the desired initial pH.

Examples of the acidic pH modifier include an inorganic acid, such as hydrochloric acid, nitric acid, and sulfuric acid, an organic acid, such as acetic acid and citric acid, and a liquid obtained by adding a cation exchange resin or the like to water, ethanol, or the like, and one or more kind selected from the group consisting of hydrochloric acid, sulfuric acid, nitric acid, and citric acid is preferred.

Examples of the alkaline pH modifier include sodium hydroxide, sodium hydrogen carbonate, potassium hydroxide, ammonium hydroxide, diethanolamine, triethanolamine, and trishydroxymethylaminomethane, and one or more kind selected from the group consisting of sodium hydroxide and ammonium hydroxide is preferred.

The pH of the emulsion liquid may have pH that is a desired value or less, and in this case, it is preferred to regulate by using the alkaline pH modifier.

The reaction temperature of the sol-gel reaction in the step I may be an optional value selected from the melting point of water contained in the water phase or more and the boiling point thereof or less, and the temperature is preferably in a certain range from the standpoint of regulating the balance between the hydrolytic decomposition reaction and the condensation reaction in the sol-gel reaction and forming the dense shell. The range is preferably 5°C or more and 60°C or less, and more preferably 10°C or more and 50°C or less.

The reaction time of the sol-gel reaction in the step I is preferably 0.5 hour or more and 50 hours or less assuming that the time when the temperature in the reaction system becomes the predetermined reaction temperature is the start of reaction.

In the case where the shell of the silica capsules is the aforementioned multilayer shell, one specific example of the silica capsules preferably contains, as a constitutional component, silica that is formed in such a manner that a tetraalkoxysilane as a silica precursor is further added to the aqueous dispersion containing the silica capsules obtained through the step I (which are hereinafter referred to as silica capsules (1)), so as to perform the sol-gel reaction in two steps. Accordingly, the silica capsules in this case are preferably produced by a method including the step 1 and the step 2 shown below.

Step 1: A step of subjecting an emulsion liquid obtained by emulsifying a water phase component containing a cationic surfactant and an oil phase component containing the organic compound and a tetraalkoxysilane, to sol-gel reaction under an acidic condition, so as to form silica capsules (1) having a core and a first shell containing silica as a constitutional component, resulting in an aqueous dispersion containing the silica capsules (1)

Step 2: A step of further adding a tetraalkoxysilane to the aqueous dispersion containing the silica capsules (1) obtained in the step 1, so as to perform sol-gel reaction, resulting in silica capsules having a second shell encapsulating the first shell

In the description herein, the expression "encapsulating the first shell" in the case where the step 1 and the step 2 are performed means encapsulating the first shell of the silica capsules (1) formed in the step 1, and also includes encapsulating the silica capsules (1).

The step 2 further forms a shell on the silica capsules formed in the step 1, and the silica capsules obtained in the step 2 become silica capsules having a shell with an increased thickness in the entirety thereof, which are considered to be silica capsules having a shell including the shell formed in the step 1 as an inner shell and the shell formed in the step 2 as an outer shell.

The step 1 may be performed in the same procedure as in the step I described above.

The amount of the tetraalkoxysilane used in the step 2 is preferably 7 parts by mass or more from the standpoint of forming the second shell encapsulating the first shell, and is also preferably 200 parts by mass or less from the standpoint of suppressing the formation of silica sol dispersed in the water phase, and enhancing the dispersion stability of the silica capsules, and the standpoint of achieving the favorable particle diameter of the microcapsules, and enhancing the particle diameter uniformity, all per 100 parts by mass of the organic compound used in the step 1.

The total amount of the tetraalkoxysilane used in the case where the step 1 and the step 2 are performed, i.e., the total amount of the tetraalkoxysilane used in the step 1 and the step 2, is preferably 30 parts by mass or more and is also preferably 250 parts by mass or less per 100 parts by mass of the organic compound used in the step 1.

The median diameter $D_{50}$ of the microcapsules as the component (A) is preferably 100 $\mu$m or less, more preferably 75 $\mu$m or less, further preferably 50 $\mu$m or less, still further preferably 30 $\mu$m or less, and still more further preferably 10 $\mu$m or less, from the standpoint of enhancing the dispersion stability of the microcapsules according to the Stokes' law, and is also preferably 0.01 $\mu$m or more, more preferably 0.05 $\mu$m or more, further preferably 0.07 $\mu$m or more, still further preferably 0.1 $\mu$m or more, still more further preferably 0.5 $\mu$m or more, and even further preferably 1 $\mu$m or more, from the standpoint of reducing the specific surface area of the microcapsules, and enhancing the retainability of the organic compound, and the standpoint of achieving the favorable particle diameter of the microcapsules, and enhancing the particle diameter uniformity. The median diameter $D_{50}$ of the component (A) can be measured according to the method shown in the examples.

The difference in specific gravity between the component (A) and the aqueous medium is preferably less than 0.30, more preferably less than 0.20, further preferably less than 0.15, still further preferably less than 0.10, still more further preferably less than 0.05, and even further preferably less than 0.01, and it is even still further preferred that there is no difference in specific gravity between them, from the standpoint of enhancing the dispersion stability of the microcapsules, and suppressing the decrease in fluidity of the aqueous microcapsule dispersion liquid with the lapse of time, and the standpoint of achieving the favorable particle diameter, and enhancing the particle diameter uniformity.

The specific gravity of the component (A) is determined by the specific gravities of the shell and the core constituting the component (A), and the mass ratio of the shell and the core.

<Component (B)>

The aqueous microcapsule dispersion liquid of the present invention contains the polysaccharide including a cationically modified branched chain structure (i.e., the cationized branched polysaccharide) as the component (B) from the standpoint of enhancing the dispersion stability of the microcapsules, and the standpoint of achieving the favorable particle diameter of the microcapsules, and enhancing the particle diameter uniformity. One kind of the cationized branched polysaccharide as the component (B) may be used alone, or two or more kinds thereof may be used in combination.

The cation charge density of the component (B) is preferably 0.3 meq/g or more, more preferably 0.5 meq/g or more, and further preferably 0.7 meq/g or more, and is also preferably 3.0 meq/g or less, more preferably 2.5 meq/g or less, further preferably 2.0 meq/g or less, still further preferably 1.7 meq/g or less, and still more further preferably 1.5 meq/g or less, from the standpoint of enhancing the dispersion stability of the microcapsules, and the standpoint of achieving the favorable particle diameter of the microcapsules, and enhancing the particle diameter uniformity.

In the present invention, the cation charge density can be calculated from the following expression (1).

$$\text{Cation charge density (meq/g)} = (\text{nitrogen content (\% by mass)}) \div 14 \times 10 \qquad (1)$$

In the expression, the nitrogen content in the component (B) can be measured by the Kjeldahl method.

In the case where two or more kinds of the cationized branched polysaccharides are used as the component (B), the cation charge density can be calculated as weighted average from the cation charge densities and the blending amounts of the cationized branched polysaccharides.

The polymer skeleton of the component (B) is preferably a polysaccharide containing hexose residues connected to each other in which at least one of the hexose residues constituting the polysaccharide has three or more bonding sites selected from the group consisting of a 1,2-bond, a 1,3-bond, a 1,4-bond, and a 1,6-bond as the bonding sites to the adjacent hexose residue, more preferably a polysaccharide containing hexose residues connected to each other in which at least one of the hexose residues constituting the polysaccharide has three bonding sites selected from the group consisting of a 1,2-bond, a 1,3-bond, a 1,4-bond, and a 1,6-bond as the bonding sites to the adjacent hexose residue, and further preferably a polysaccharide having a main chain containing hexose residues connected to each other via a 1,4-bond, and a branched chain containing a hexose residue branched from the hexose residue of the main chain via a 1,6-bond.

The number of the hexose residue per one branched chain may be one, or may be two or more.

Preferred examples of the hexose constituting the polymer skeleton of the component (B) include one or more kind selected from the group consisting of mannose, galactose, glucose, and xylose.

Preferred examples of the polymer skeleton of the component (B) include one or more kind selected from the group consisting of a polysaccharide having a main chain containing mannose residues connected to each other via a $\beta$-1,4-bond, and a branched chain containing a galactose residue branched from the mannose residue of the main chain via an $\alpha$-1,6-bond, and a polysaccharide having a main chain containing glucose residues connected to each other via an $\alpha$-1,4-bond, and a branched chain containing a glucose residue branched from the glucose residue of the main chain via an $\alpha$-1,6-bond.

In the case where the component (B) is a mixture containing the polysaccharide having a cationically modified branched chain structure and the polysaccharide having a cationically modified linear chain structure, it suffices that the polysaccharide having a cationically modified branched chain structure contained as a constitutional component of the component (B) has the aforementioned polymer skeleton.

Specific examples of the component (B) include cationized galactomannan, such as cationized guar gum, cationized tara gum, cationized fenugreek gum, and cationized locust bean gum; cationized starch; and tamarind seed gum.

Among these, the component (B) is preferably one or more kind selected from the group consisting of cationized galactomannan and cationized starch, and more preferably cationized galactomannan, from the standpoint of enhancing the dispersion stability of the microcapsules, the standpoint of achieving the favorable particle diameter of the microcapsules, and enhancing the particle diameter uniformity, and the standpoint of enabling the enhancement of the dispersion stability of the microcapsules even with a small amount thereof.

The cationized galactomannan is a modified polysaccharide including galactomannan having a main chain containing mannose as a constitutional unit, and a side chain containing galactose as a constitutional unit, having a quaternary ammonium group introduced thereto. Galactomannan can be obtained, for example, from endosperm of legume seeds.

It is considered that the cationized galactomannan has multiple hydroxy groups and cationic groups, and simultaneously has a branched chain structure, and thereby the steric repulsive force and the electrostatic repulsive force can more effectively act among the microcapsules to suppress the formation of aggregates of the microcapsules, resulting in the enhancement of the dispersion stability of the microcapsules, the achievement of the favorable particle diameter of the microcapsules, and the enhancement of the particle diameter uniformity. From this standpoint, the component (B) is further preferably one or more kind selected from the group consisting of cationized guar gum, cationized tara gum, cationized fenugreek gum, and cationized locust bean gum, still further preferably one or more kind selected from the group consisting of cationized guar gum and cationized tara gum, still more further preferably cationized guar gum, and even further preferably one or more kind selected from the group consisting of O-(2-hydroxy-3-(trimethylammonio)propyl) guar gum chloride (label name: guar hydroxypropyltrimonium chloride) and a hydroxypropyl derivative of O-(2-hydroxy-3-(trimethylammonio)propyl) guar gum chloride (label name: hydroxypropyl guar hydroxypropyltrimonium chloride).

Examples of the commercially available product of the component (B) include cationized guar gum, such as Jaguar Series, available from Solvay S.A., and Rhaball Gum Series, available from DSP Gokyo Food & Chemical Co., Ltd.; cationized tara gum, such as Catinal CTR-100, available from Toho Chemical Industry, Co., Ltd.; cationized locust bean gum, such as Catinal CLB-100, available from Toho Chemical Industry, Co., Ltd.; and cationized starch, such as Excell Series, available from Nippon Starch Chemical Co., Ltd.

The aqueous microcapsule dispersion liquid of the present invention may contain any other component than the component (A) and the component (B) depending on necessity. Examples of the other component include a pH modifier, a colorant, an antiseptic, an antioxidant, an ultraviolet ray absorbent, a shell surface modifier, an inorganic salt, a thickener, a deposition aid, and a rheology modifier.

The aqueous microcapsule dispersion liquid of the present invention may be allowed to contain in advance a fabric softener, a fabric freshener, a fabric toughening agent, an enzyme, a builder liquid, a hair conditioner, a skin conditioner, a fragrance material, clay, zeolite, silicone, and the like, other than the component (A) and the component (B), depending on necessity, for blending the aqueous dispersion liquid in various formulations.

(Production of Aqueous Microcapsule Dispersion Liquid)

The method of producing the aqueous microcapsule dispersion liquid of the present invention is not particularly limited. For example, the aqueous microcapsule dispersion liquid can be produced by a method including a step of mixing the component (A) which have been produced by known methods in advance, the component (B), and depending on necessity the aforementioned other component. In particular, the method of producing the aqueous microcapsule dispersion liquid of the present invention is preferably a method including a step of mixing an aqueous dispersion containing the component (A) and the component (B), and depending on necessity the aforementioned other component from the standpoint of the easiness of production.

In the case where the component (A) is silica capsules, the method of producing the aqueous microcapsule dispersion liquid of the present invention is preferably a method including a step of mixing an aqueous dispersion containing silica capsules as the component (A) and the component (B), and depending on necessity the aforementioned other component. The aqueous dispersion containing silica capsules can be obtained by the method described above.

In the present invention, the mixing method of the aqueous dispersion containing the component (A) and the component (B) is not particularly limited, and preferably includes a step of mixing by adding the component (B) to the aqueous dispersion containing the component (A) from the standpoint of the easiness of production.

The component (B) may be used in the form of an aqueous solution from the standpoint of the easiness of blending. The aqueous solution of the component (B) may be obtained by dissolving the component (B) in water while regulating the pH corresponding to the solubility of the component (B) in water.

The mixing temperature of the aqueous dispersion containing the component (A) and the component (B), and depending on necessity the aforementioned other component is preferably 15°C or more, and more preferably 20°C or more, and is also preferably 35°C or less, and more preferably 30°C or less, from the standpoint of the dispersion stability of the microcapsules.

The components may be mixed by using a known agitation device or the like.

(Composition of Aqueous Microcapsule Dispersion Liquid)

The content or the blending amount of the component (A) in the aqueous microcapsule dispersion liquid of the present invention is preferably 50% by mass or less, more preferably 40% by mass or less, further preferably 30% by mass or less, and still further preferably 25% by mass or less, from the standpoint of reducing the viscosity of the aqueous dispersion liquid, and enhancing the handleability, the standpoint of enhancing the dispersion stability of the microcapsules, and the standpoint of achieving the favorable particle diameter of the microcapsules, and enhancing the particle diameter uniformity, and is also preferably 3% by mass or more, more preferably 5% by mass or more, further preferably 10% by mass or more, still further preferably 15% by mass or more, still more further preferably 18% by mass or more, and even further preferably 20% by mass or more, from the standpoint of facilitating the preparation of a liquid composition or a product using the aqueous dispersion liquid.

The content or the blending amount of the component (B) in the aqueous microcapsule dispersion liquid of the present invention is preferably 0.001% by mass or more, more preferably 0.005% by mass or more, further preferably 0.01% by mass or more, still further preferably 0.03% by mass or more, and still more further preferably 0.05% by mass or more, from the standpoint of the dispersion stability of the microcapsules, and the standpoint of achieving the favorable particle diameter of the microcapsules, and enhancing the particle diameter uniformity, and is also preferably 1.5% by mass or less, more preferably 1% by mass or less, further preferably 0.7% by mass or less, still further preferably 0.5% by mass or less, still more further preferably 0.3% by mass or less, even further preferably 0.2% by mass or less, even still further preferably 0.15% by mass or less, even still more further preferably 0.13% by mass or less, and yet further preferably 0.1% by mass or less, from the standpoint of suppressing the formation of aggregates of the microcapsules in the aqueous dispersion liquid, and enhancing the dispersion stability of the microcapsules, the standpoint of achieving the favorable particle diameter of the microcapsules, and enhancing the particle diameter uniformity, and the standpoint of reducing the viscosity of the aqueous dispersion liquid, and enhancing the handleability.

The content or the blending amount of the component (B) per 100 parts by mass of the component (A) in the aqueous microcapsule dispersion liquid of the present invention is preferably 0.03 part by mass or more, more preferably 0.05 part by mass or more, further preferably 0.1 part by mass or more, still further preferably 0.15 part by mass or more, and still more further preferably 0.2 part by mass or more, from the standpoint of the dispersion stability of the microcapsules, and the standpoint of achieving the favorable particle diameter of the microcapsules, and enhancing the particle diameter uniformity, and is also preferably 3 parts by mass or less, more preferably 2 parts by mass or less, further preferably 1 part by mass or less, still further preferably 0.8 part by mass or less, and still more further preferably 0.6 part by mass or less, from the standpoint of suppressing the formation of aggregates of the microcapsules in the aqueous dispersion liquid, and enhancing the dispersion stability of the microcapsules, the standpoint of achieving the favorable particle diameter of the microcapsules, and enhancing the particle diameter uniformity, and the standpoint of reducing the viscosity of the aqueous dispersion liquid, and enhancing the handleability.

The content or the blending amount of water in the aqueous microcapsule dispersion liquid of the present invention is preferably 50% by mass or more, more preferably 60% by mass or more, further preferably 70% by mass or more, and still further preferably 75% by mass or more, and is also preferably 97% by mass or less, more preferably 95% by mass or less, further preferably 90% by mass or less, and still further preferably 85% by mass or less, from the standpoint of the dispersion stability of the microcapsules, and the standpoint of achieving the favorable particle diameter of the microcapsules, and enhancing the particle diameter uniformity.

The viscosity at 25°C of the aqueous microcapsule dispersion liquid of the present invention is preferably 1 mPa·s or more, more preferably 3 mPa·s or more, further preferably 5 mPa·s or more, and still further preferably 10 mPa·s or more, and is also preferably 4,000 mPa s or less, more preferably 2,000 mPa s or less, further preferably 1,000 mPa s or less, still further preferably 500 mPa s or less, still more further preferably 100 mPa s or less, even further preferably 50 mPa·s or less, even still further preferably 30 mPa·s or less, even still more further preferably 20 mPa·s or less.

The viscosity at 25°C of the aqueous microcapsule dispersion liquid of the present invention can be measured by using a B-type viscometer (Model: TVB-10, available from Toki Sangyo Co., Ltd.) with a spindle of M3 or M4 at a rotation speed of 60 rpm or 6 rpm and a measurement temperature of 25°C, as described in the examples.

The aqueous microcapsule dispersion liquid of the present invention is excellent in dispersion stability of the microcapsules, has a favorable particle diameter of the microcapsules, is excellent in particle diameter uniformity, and is excellent in handleability, and therefore can be used in various applications. Examples of the applications include perfumery and cosmetics, such as an emulsion lotion, a cosmetic liquid, a cosmetic lotion, a beauty lotion, a cosmetic cream, a gel formulation, a hair treatment, and a quasi drug; a fiber treatment agent, such as a detergent, a softener, and an anti-wrinkle spray; a sanitary product, such as a disposable diaper; and a perfuming agent, and the aqueous microcapsule dispersion liquid can be favorably applied to the production in these applications.

The aqueous microcapsule dispersion liquid of the present invention is preferably used by being contained or by blending in a liquid composition, such as a detergent composition, a fiber treatment agent composition, a perfumery and cosmetic composition, a perfuming agent composition, and a deodorizer composition. The composition is preferably one or more kind selected from the group consisting of a detergent composition, such as a powder detergent composition and a liquid detergent composition; and a fiber treatment agent composition, such as a softener composition, more preferably a fiber treatment agent composition, and further preferably a softener composition.

In relation to the embodiments described above, the present invention further relates to the aqueous microcapsule dispersion liquids and the methods of producing an aqueous microcapsule dispersion liquid shown below.

<1> An aqueous microcapsule dispersion liquid containing a component (A) and a component (B) below:

component (A): microcapsules having a shell containing an inorganic material as a constitutional component, and a core containing one or more kind of an organic compound, inside the shell, and

component (B): a polysaccharide including a cationically modified branched chain structure.

<2> The aqueous microcapsule dispersion liquid according to the item <1>, wherein the component (B) has a cation charge density of preferably 0.3 meq/g or more, more preferably 0.5 meq/g or more, and further preferably 0.7 meq/g or more, and of preferably 3.0 meq/g or less, more preferably 2.5 meq/g or less, further preferably 2.0 meq/g or less, still further preferably 1.7 meq/g or less, and still more further preferably 1.5 meq/g or less.

<3> The aqueous microcapsule dispersion liquid according to the item <1> or <2>, wherein the component (B) is preferably one or more kind selected from the group consisting of cationized galactomannan and cationized starch, more preferably cationized galactomannan, further preferably cationized guar gum, still further preferably one or more kind selected from the group consisting of O-(2-hydroxy-3-(trimethylammonio)propyl) guar gum chloride (label name: guar hydroxypropyltrimonium chloride) and a hydroxypropyl derivative of O-(2-hydroxy-3-(trimethylammonio)propyl) guar gum chloride (label name: hydroxypropyl guar hydroxypropyltrimonium chloride).

<4> The aqueous microcapsule dispersion liquid according to any one of the items <1> to <3>, wherein the inorganic material constituting the shell of the component (A) is preferably a metal oxide containing a metal element or a semimetal element, more preferably an inorganic polymer formed through sol-gel reaction using a metal alkoxide $(M(OR)_x)$ as a shell precursor, further preferably an inorganic polymer formed through sol-gel reaction using a metal alkoxide of one or more kind selected from the group consisting of silicon, aluminum, and titanium as a shell precursor, and still further preferably silica formed through sol-gel reaction using an alkoxysilane as a shell precursor.

<5> The aqueous microcapsule dispersion liquid according to the item <4>, wherein the alkoxysilane is preferably a tetraalkoxysilane.

<6> The aqueous microcapsule dispersion liquid according to any one of the items <1> to <3>, wherein the component (A) is microcapsules having a shell containing silica as a constitutional component, and a core containing one or more kind of an organic compound, inside the shell.

<7> The aqueous microcapsule dispersion liquid according to the item <6>, wherein the shell is a multilayer shell including an inner shell containing silica, which is a hydrolytic polycondensation product of an alkoxysilane, as a constitutional component, and an outer shell containing silica, which is a hydrolytic polycondensation product of an alkoxysilane, as a constitutional component, outside the inner shell.

<8> The aqueous microcapsule dispersion liquid according to any one of the items <1> to <7>, wherein the organic compound is preferably one or more kind selected from the group consisting of a fragrance material; a fragrance precursor; an oil; an antioxidant; an antibacterial agent; a fertilizer; a surface modifier for fibers, skin, hair, and the like; a cooling agent; a dye; a colorant; a silicone; a solvent; and an oil soluble polymer, more preferably one or more kind selected from the group consisting of a fragrance material, a fragrance precursor, an oil, an antioxidant, an antibacterial agent, a fertilizer, a surface modifier, and a solvent, further preferably one or more kind selected from the group consisting of a fragrance material, a fragrance precursor, an oil, an antioxidant, and a solvent, still further preferably one or more kind selected from the group consisting of a fragrance material, a fragrance precursor, and an oil, and still more further preferably one or more kind selected from the group consisting of a fragrance material and a fragrance precursor.

<9> The aqueous microcapsule dispersion liquid according to any one of the items <1> to <8>, wherein the organic compound has a cLogP value of preferably 1 or more, more preferably 2 or more, further preferably 3 or more, and still further preferably 4 or more, and of preferably 30 or less, more preferably 20 or less, and further preferably 10 or less.

<10> The aqueous microcapsule dispersion liquid according to any one of the items <1> to <9>, wherein the microcapsules as the component (A) has a median diameter $D_{50}$ of preferably 100 μm or less, more preferably 75 μm or less, further preferably 50 μm or less, still further preferably 30 μm or less, and still more further preferably 10 μm or less, and of preferably 0.01 μm or more, more preferably 0.05 μm or more, further preferably 0.07 μm or more, still further preferably 0.1 μm or more, still more further preferably 0.5 μm or more, and even further preferably 1 μm or more.

<11> An aqueous microcapsule dispersion liquid containing a component (A) and a component (B) below:

component (A): microcapsules having a shell containing silica as a constitutional component, and a core containing one or more kind of an organic compound, inside the shell, and

component (B): one or more kind selected from the group consisting of cationized galactomannan and cationized starch.

<12> The aqueous microcapsule dispersion liquid according to the item <11>, wherein the component (B) has a cation charge density of preferably 0.3 meq/g or more, more preferably 0.5 meq/g or more, and further preferably 0.7 meq/g or more, and of preferably 3.0 meq/g or less, more preferably 2.5 meq/g or less, further preferably 2.0 meq/g or less, still further preferably 1.7 meq/g or less, and still more further preferably 1.5 meq/g or less.

<13> The aqueous microcapsule dispersion liquid according to the item <11> or <12>, wherein the component (B) is preferably cationized galactomannan, more preferably one or more kind selected from the group consisting of cationized guar gum, cationized tara gum, cationized fenugreek gum, and cationized locust bean gum, further preferably one or more kind selected from the group consisting of cationized guar gum and cationized tara gum, still further preferably cationized guar gum, still more further preferably one or more kind selected from the group consisting of O-(2-hydroxy-3-(trimethylammonio)propyl) guar gum chloride (label name: guar hydroxypropyltrimonium chloride) and a hydroxypropyl derivative of O-(2-hydroxy-3-(trimethylammonio)propyl) guar gum chloride (label name: hydroxypropyl guar hydroxypropyltrimonium chloride).

<14> The aqueous microcapsule dispersion liquid according to any one of the items <1> to <13>, wherein a content or a blending amount of the component (A) in the aqueous microcapsule dispersion liquid is preferably 50% by mass or less, more preferably 40% by mass or less, further preferably 30% by mass or less, and still further preferably 25% by mass or less, and is also preferably 3% by mass or more, more preferably 5% by mass or more, further preferably 10% by mass or more, still further preferably 15% by mass or more, still more further preferably 18% by mass or more, and even further preferably 20% by mass or more.

<15> The aqueous microcapsule dispersion liquid according to any one of the items <1> to <14>, wherein a content or a blending amount of the component (B) in the aqueous microcapsule dispersion liquid is preferably 0.001% by mass or more, more preferably 0.005% by mass or more, further preferably 0.01% by mass or more, still further preferably 0.03% by mass or more, and still more further preferably 0.05% by mass or more, and is also preferably 1.5% by mass or less, more preferably 1% by mass or less, further preferably 0.7% by mass or less, still further preferably 0.5% by mass or less, still more further preferably 0.3% by mass or less, even further preferably 0.2% by mass or less, even still further preferably 0.15% by mass or less, even still more further preferably 0.13% by mass or less, and yet further preferably 0.1% by mass or less.

<16> The aqueous microcapsule dispersion liquid according to any one of the items <1> to <15>, wherein a content or a blending amount of the component (B) per 100 parts by mass of the component (A) in the aqueous microcapsule dispersion liquid is preferably 0.03 part by mass or more, more preferably 0.05 part by mass or more, further preferably 0.1 part by mass or more, still further preferably 0.15 part by mass or more, and still more further preferably 0.2 part by mass or more, and is also preferably 3 parts by mass or less, more preferably 2 parts by mass or less, further preferably 1 part by mass or less, still further preferably 0.8 part by mass or less, and still more further preferably 0.6 part by mass or less.

<17> A method of producing the aqueous microcapsule dispersion liquid according to any one of the items <1> to <16>, including a step of mixing an aqueous dispersion containing the component (A) and the component (B).

Examples

The measurements in Examples and Comparative Examples were performed by the following methods.

[Median Diameter $D_{50}$]

The median diameter $D_{50}$ of the emulsion droplets and the median diameter $D_{50}$ of the microcapsules were measured by using a laser diffraction/scattering particle diameter distribution measuring device, "LA-960" (trade name, available from Horiba, Ltd.). In the measurement, a flow cell was used, water was used as a medium, and the refractive index of the dispersoid was set to 1.45-0i. The emulsion liquid or the aqueous dispersion containing the microcapsules was added to the flow cell, and measured at a concentration showing a transmittance of approximately 90%, so as to obtain the volume based median diameter $D_{50}$.

<Model Fragrance Material>

As the organic compound encapsulated in the microcapsules, a model fragrance material A (volume average cLogP: 3.8, specific gravity: 0.88) having the composition shown in Table 1 was used. The volume average cLogP value of the model fragrance material was calculated in such a manner that the cLogP values of all the fragrance components contained in the model fragrance material were multiplied by the volume proportions thereof in the model fragrance material respectively, and the resulting values were summated.

Table 1: Model Fragrance Material A

| Name of fragrance component | Content (% by mass) | cLogP |
|---|---|---|
| Linalool | 22.0 | 3.3 |
| Linalyl acetate | 16.2 | 4.4 |
| Tetrahydrolinalool | 16.0 | 3.6 |
| Caryophyllene | 4.5 | 6.3 |
| Coumarin | 4.0 | 1.5 |
| 1,8-Cineole | 3.1 | 3.1 |
| Isobornyl acetate | 3.0 | 3.9 |
| Ocimene | 2.9 | 4.8 |
| Borneol | 2.6 | 2.9 |
| Neryl acetate | 2.2 | 4.5 |
| Others | 23.5 | |

(Synthesis of Component (A))

Synthesis Example 1

(Step 1)

3.00 g of Quartamin 60W (trade name, cetyltrimethylammonium chloride, active ingredient: 30% by mass, available from Kao Corporation) was diluted with 747.00 g of ion exchanged water to provide a water phase component. An oil phase component prepared by mixing 200.70 g of the model fragrance material A and 50.08 g of tetraethoxysilane (which may be hereinafter referred to as "TEOS") was added to the water phase component, and the mixed liquid was emulsified at room temperature (approximately 25°C) for 15 minutes with a homomixer (Model HM-310, available from HsiangTai Machinery Industry Co., Ltd.) set to a rotation number of 8,500 rpm and 15 minutes, so as to provide a emulsion liquid. The median diameter $D_{50}$ of the emulsion droplets at this time was 1.1 μm.

Subsequently, the resulting emulsion liquid was regulated for the pH thereof to 3.8 with a 1% by mass sulfuric acid aqueous solution, then transferred to a separable flask equipped with an agitation blade and a condenser, and agitated at 200 rpm for 24 hours while retaining the liquid temperature to 30°C, so as to provide an aqueous dispersion containing silica capsules having a core formed of the model fragrance material A and a first shell.

(Step 2)

Subsequently, 32.8 g of TEOS was added to 973.24 g of the aqueous dispersion obtained in the step 1 in a dropwise manner over 420 minutes. After the dropwise addition, the mixture was further agitated for 17 hours to form a second shell encapsulating the first shell, resulting in an aqueous dispersion containing silica capsules (A-1) having the model fragrance material A encapsulated by a multilayer shell including an inner shell containing silica as a hydrolytic polycondensation product of an alkoxysilane, as a constitutional component, and an outer shell containing silica as a hydrolytic polycondensation product of an alkoxysilane, as a constitutional component, outside the inner shell, in 21.7% by mass. The median diameter $D_{50}$ of the silica capsules (A-1) was 2.2 μm. The content of the silica capsules (A-1) in the aqueous dispersion described above is a calculated value from the blending formulation in synthesizing the silica capsules.

(Production of Aqueous Microcapsule Dispersion Liquid)

Examples 1 and 2 and Comparative Examples 1 to 3

The aqueous dispersion containing the component (A) obtained in Synthesis Example 1 and an aqueous solution of the component (B) were mixed at room temperature (approximately 25°C) to make the contents of the component (A) and the component (B) in the aqueous dispersion liquid shown in Table 2 below, resulting in an aqueous microcapsule dispersion liquid.

The aqueous solution of the component (B) used was obtained by dissolving the powder material of the component (B) in ion exchanged water to make the prescribed concentration depending on necessity. In Examples 1 and 2, the aqueous solution of the component (B) used was regulated for the pH thereof to 4 to 5 with a 1% by mass sulfuric acid aqueous solution.

In Comparative Example 1, the component (B) was not used, and the aqueous dispersion containing the silica capsules (A-1) obtained in Synthesis Example 1 was subjected directly to the evaluation of dispersion stability.

The cationized branched polysaccharides and the polymer dispersants used in Examples and Comparative Examples in Table 2 are shown below.

Hydroxypropyl guar hydroxypropyltrimonium chloride: Jaguar C-162 (cationized guar gum (hydroxypropyl derivative of O-(2-hydroxy-3-(trimethylammonio)propyl) guar gum chloride), cation charge density: 0.93 meq/g, available from Solvay S.A., appearance: powder)

Guar hydroxypropyltrimonium chloride: Jaguar C-17K (cationized guar gum (O-(2-hydroxy-3-(trimethylammonio)propyl) guar gum chloride), cation charge density: 1.07 to 1.50 meq/g, available from Solvay S.A., appearance: powder)

Hydroxyethyl cellulose: Natrosol 25011HR PC (available from Ashland, Inc., appearance: powder)

Dimethyldiallyl ammonium chloride-acrylamide copolymer: Merquat 550 (active ingredient: 9%) (available from Lubrizol Japan, Ltd.)

In Table 2, the content of the component (A) is a calculated value from the blending amount of the aqueous dispersion containing the component (A) obtained in Synthesis Example 1, the content of the component (A) in the aqueous dispersion, the blending amount of the aqueous solution of the component (B), and the content of the component (B) in the aqueous solution.

[Evaluation of Dispersion Stability of Microcapsules]

The aqueous microcapsule dispersion liquid obtained in each of Examples 1 and 2 and Comparative Examples 1 to 3 was allowed to stand at room temperature (approximately 25°C), and the dispersion stability of the microcapsules in the aqueous dispersion liquid was evaluated by visually observing presence or absence of the decrease of fluidity (caking) and the aggregation of the aqueous dispersion liquid. In Table 2, the case shown by "> X days" means that both the decrease of fluidity and the aggregation of the microcapsules were not confirmed at least within X days, and the case shown by "< X days" means that the decrease of fluidity or the aggregation of the microcapsules was confirmed on the Xth day.

A lager number of days within which both the decrease of fluidity and the aggregation of the microcapsules are not confirmed means better dispersion stability of the microcapsules in the aqueous dispersion liquid.

In Examples 1 and 2, a white turbid phase was confirmed while gradually separating into a white turbid phase and a transparent phase, but no solidified phase was confirmed, and it was confirmed that the fluidity was retained after storing.

30 mL of the aqueous microcapsule dispersion liquid after standing still of each of Examples 1 and 2 was sealed in a glass vial (capacity: 50 mL), and agitated with a magnetic stirrer at room temperature (approximately 25°C) for approximately 5 minutes, and thereby the white turbid phase returned to the original dispersed state, and the decrease of fluidity and the aggregation were not confirmed by visual observation after the agitation. 30 mL of the aqueous microcapsule dispersion liquid after standing still of each of Examples 1 and 2 was sealed in a glass vial (capacity: 50 mL), and vigorously shaken by hand approximately 20 times, and thereby the white turbid phase returned to the original dispersed state, and the decrease of fluidity and the aggregation were not confirmed by visual observation after the shaking. It is estimated therefrom that the aqueous microcapsule dispersion liquids obtained in Examples 1 and 2 each are flocculate of microcapsules since it can return to the original dispersed state through agitation or shaking even after storing, and the decrease of fluidity and the aggregation are not confirmed by visual observation after agitation or shaking.

Table 2

| | | Aqueous microcapsule dispersion liquid | | | | | Evaluation |
|---|---|---|---|---|---|---|---|
| | | Component (A) | | Component (B) | | Content (blending amount) of component (B) per 100 parts by mass of component (A) (part by mass) | Dispersion stability (storing temperature: room temperature) |
| | | Kind | Content (Blending amount) | Kind | Content (Blending amount) | | |
| | | | (% by mass) | | (% by mass) | | |
| Example | 1 | silica capsules (A-1) | 19.5 | hydroxypropyl guar hydroxypropyltrimonium chloride | 0.10 | 0.51 | > 14 days |
| | 2 | silica capsules (A-1) | 20.6 | guar hydroxypropyltrimonium chloride | 0.05 | 0.24 | > 14 days |
| Comparative Example | 1 | silica capsules (A-1) | 21.7 | - | - | - | < 4 days |
| | 2 | silica capsules (A-1) | 19.4 | hydroxyethyl cellulose | 0.11 | 0.57 | < 4 days |
| | 3 | silica capsules (A-1) | 20.2 | dimethyldiallyl ammonium chloride-acrylamide copolymer | 0.62 | 3.07 | < 6 days |

It is understood from Table 2 that the aqueous microcapsule dispersion liquids of Examples each are excellent in dispersion stability as compared to Comparative Examples. Comparative Example 3 contains a synthetic polymer having a cationic group, but does not provide good dispersion stability.

Consequently, the present invention can enhance the dispersion stability of the microcapsules in the aqueous dispersion liquid.

(Production of Aqueous Microcapsule Dispersion Liquid)

Examples 3 to 5 and Comparative Example 4

The aqueous dispersion containing the component (A) obtained in Synthesis Example 1 and an aqueous solution of the component (B) were mixed at room temperature (approximately 25°C) to make the contents of the component (A) and the component (B) in the aqueous dispersion liquid shown in Table 3 below, resulting in an aqueous microcapsule dispersion liquid.

The aqueous solution of the component (B) used was obtained by dissolving the powder material of the component (B) in ion exchanged water to make the prescribed concentration depending on necessity. In Example 3, the aqueous solution of the component (B) used was regulated for the pH thereof to 4 to 5 with a 1% by mass sulfuric acid aqueous solution.

The cationically modified polysaccharides and the polymer dispersants used in Examples 3 to 5 and Comparative Example 4 in Table 3 are shown below.

Hydroxypropyl guar hydroxypropyltrimonium chloride: Jaguar C-162 (cationized guar gum (hydroxypropyl derivative of O-(2-hydroxy-3-(trimethylammonio)propyl) guar gum chloride), cation charge density: 0.93 meq/g, available from Solvay S.A., appearance: powder)

Cationized tara gum: Catinal CTR-100 (available from Toho Chemical Industry, Co., Ltd., appearance: powder)

Cationized starch: Excell DH (available from Nippon Starch Chemical Co., Ltd., appearance: powder)

Cationized HEC: Poiz C-60H (Polyquaternium-10, O-(2-hydroxy-3-(trimethylammonio)propyl) hydroxyethyl cellulose, cation charge density: 1.1 to 1.8 meq/g, available from Kao Corporation, appearance: powder (effective ingredient: 90%))

In Table 3, the content of the component (A) is a calculated value from the blending amount of the aqueous dispersion containing the component (A) obtained in Synthesis Example 1, the content of the component (A) in the aqueous dispersion, the blending amount of the aqueous solution of the component (B), and the content of the component (B) in the aqueous solution.

[Evaluation of Dispersion Stability of Microcapsules]

The aqueous microcapsule dispersion liquid obtained in each of Examples 3 to 5 and Comparative Example 4 was allowed to stand at room temperature (approximately 25°C), and the dispersion stability of the microcapsules in the aqueous dispersion liquid was evaluated by visually observing presence or absence of the decrease of fluidity (caking) and the aggregation of the aqueous dispersion liquid. In Table 3, the case shown by "> X days" means that both the decrease of fluidity and the aggregation of the microcapsules were not confirmed at least within X days, and the case shown by "< X days" means that the decrease of fluidity or the aggregation of the microcapsules was confirmed on the Xth day.

A lager number of days within which both the decrease of fluidity and the aggregation of the microcapsules are not confirmed means better dispersion stability of the microcapsules in the aqueous dispersion liquid.

[Evaluation of Particle Diameter Uniformity of Microcapsules]

The aqueous microcapsule dispersion liquid obtained in each of Examples 2 to 5 and Comparative Example 4 was stored by allowing to stand at room temperature (approximately 25°C) for 14 days, and then the microcapsules contained in the aqueous dispersion liquid after storing were measured for the volume based median diameter $D_{50}$ in the same manner as in the measurement of the median diameter $D_{50}$ of the microcapsules described above by using a laser diffraction/scattering particle diameter distribution measuring device, "LA-960" (trade name, available from Horiba, Ltd.). The results are shown in Table 3.

The coefficient of variation CV was calculated according to the following expression from the standard variation and the average value in the volume based particle diameter distribution of the microcapsules contained in the aqueous dispersion liquid after storing obtained by using the laser diffraction/scattering particle diameter distribution measuring device described above. The results are shown in Table 3. A smaller value of the coefficient of variation CV means better particle diameter uniformity.

$$\text{Coefficient of variation CV (\%)} = (\text{standard variation/average value}) \times 100$$

[Viscosity of Aqueous Microcapsule Dispersion Liquid after storing]

The aqueous microcapsule dispersion liquid obtained in each of Examples 2 to 5 and Comparative Example 4 was stored by allowing to stand at room temperature (approximately 25°C) for 14 days, and then the aqueous microcapsule dispersion liquid after storing was measured for the viscosity at 25°C by using a B-type viscometer (Model: TVB-10, available from Toki Sangyo Co., Ltd.) with a spindle of M3 or M4 at a rotation speed of 60 rpm or 6 rpm and a measurement temperature of 25°C. The results are shown in Table 3.

Table 3

| | | Aqueous microcapsule dispersion liquid | | | | | Evaluation | | | |
| | | Component (A) | | Component (B) | | Content (blending amount) of component (B) per 100 parts by mass of component (A) (part by mass) | Dispersion stability (storing temperature: room temperature) | Median diameter $D_{50}$ (storing temperature: room temperature) ($\mu$m) | Coefficient of variation CV (storing temperature: room temperature) (%) | Viscosity of aqueous microcapsule dispersion liquid after storing (mPa·s) |
| | | Kind | Content (Blending amount) (% by mass) | Kind | Content (Blending amount) (% by mass) | | | | | |
| Example | 2 | silica capsules (A-1) | 20.6 | guar hydroxypropyltrimonium chloride | 0.05 | 0.24 | > 14 days | 2.10 | 52 | 13.78 |
| | 3 | silica capsules (A-1) | 20.6 | hydroxypropyl guar hydroxypropyltrimonium chloride | 0.05 | 0.24 | > 14 days | 2.34 | 49 | 10.45 |
| | 4 | silica capsules (A-1) | 20.6 | cationized tara gum | 0.05 | 0.24 | > 14 days | 2.21 | 47 | 8.11 |
| | 5 | silica capsules (A-1) | 20.5 | cationized starch | 0.05 | 0.24 | < 14 days | 2.26 | 52 | 3.62 |
| Comparative Example | 4 | silica capsules (A-1) | 20.6 | cationized HEC | 0.05 | 0.24 | > 14 days | 5.03 | 221 | 15.71 |

It is understood from Table 3 that the aqueous microcapsule dispersion liquids of Examples each are excellent in dispersion stability, and are good in the particle diameter of the microcapsules and the viscosity of the aqueous microcapsule dispersion liquid even after storing, as compared to Comparative Example. It is understood that the aqueous microcapsule dispersion liquids of Examples each have smaller values of the coefficient of variation CV than Comparative Example, and thus each have a small variation of the particle diameter thereof, and are excellent in particle diameter uniformity. It is estimated therefrom that the aqueous microcapsule dispersion liquids of Examples each contain the cationized branched polysaccharide having a branched chain structure as the component (B), and therefore are less likely to undergo the crosslinking aggregation, and can suppress the formation of aggregates of the microcapsules, resulting in excellent particle diameter uniformity even after storing. On the other hand, it is estimated that the cationized polysaccharide contained in the aqueous microcapsule dispersion liquid of Comparative Example has a linear chain structure, and therefore the microcapsules are more likely to undergo the crosslinking aggregation, and fail to suppress the formation of aggregates of the microcapsules, resulting in inferior particle diameter uniformity.

Consequently, the present invention can enhance the dispersion stability of the microcapsules in the aqueous dispersion liquid, can provide a favorable particle diameter of the microcapsules, and can enhance the particle diameter uniformity thereof.

Industrial Applicability

The aqueous microcapsule dispersion liquid of the present invention can be stored in the state where the dispersion stability of the microcapsules is favorably retained, and furthermore has a favorable particle diameter of the microcapsules and is excellent in particle diameter uniformity of the microcapsules, even after long-term storage thereof. Accordingly, the aqueous microcapsule dispersion liquid of the present invention is free from thickening and the like due to gelation or formation of aggregates and has good handleability in imparting various functions, for example, imparting fragrance, to various products, such as a laundry product, a personal care product, a cosmetic product, and a household liquid product, and therefore can be widely utilized.

**Claims**

1. An aqueous microcapsule dispersion liquid comprising a component (A) and a component (B) below:

   component (A): microcapsules having a shell comprising an inorganic material as a constitutional component, and a core comprising one or more kind of an organic compound, inside the shell, and
   component (B): a polysaccharide comprising a cationically modified branched chain structure.

2. The aqueous microcapsule dispersion liquid according to claim 1, wherein the component (B) has a cation charge density of 0.3 meq/g or more and 3.0 meq/g or less.

3. The aqueous microcapsule dispersion liquid according to claim 1 or 2, wherein the component (B) is cationized galactomannan.

4. The aqueous microcapsule dispersion liquid according to any one of claims 1 to 3, wherein the component (B) is cationized guar gum.

5. The aqueous microcapsule dispersion liquid according to any one of claims 1 to 4, wherein a content of the component (B) per 100 parts by mass of the component (A) in the aqueous microcapsule dispersion liquid is 0.03 part by mass or more and 3 parts by mass or less.

6. The aqueous microcapsule dispersion liquid according to any one of claims 1 to 5, wherein a content of the component (A) in the aqueous microcapsule dispersion liquid is 5% by mass or more and 50% by mass or less.

7. The aqueous microcapsule dispersion liquid according to any one of claims 1 to 6, wherein the component (A) is microcapsules having a shell comprising silica as a constitutional component, and a core comprising one or more kind of an organic compound, inside the shell.

8. The aqueous microcapsule dispersion liquid according to any one of claims 1 to 7, wherein the microcapsules as the component (A) have a median diameter $D_{50}$ of 0.1 $\mu$m or more and 50 $\mu$m or less.

9. The aqueous microcapsule dispersion liquid according to any one of claims 1 to 8, wherein the organic compound is one or more kind selected from the group consisting of a fragrance material, a fragrance precursor, an oil, an antioxidant, and a solvent.

10. A method of producing the aqueous microcapsule dispersion liquid according to any one of claims 1 to 9, comprising mixing an aqueous dispersion comprising the component (A) and the component (B).

**EP 4 364 813 A1**

<table>
<tr><td colspan="3"><b>INTERNATIONAL SEARCH REPORT</b></td><td colspan="2">International application No.<br><b>PCT/JP2022/026083</b></td></tr>
</table>

**A.    CLASSIFICATION OF SUBJECT MATTER**

*A61Q 1/00*(2006.01)i; *A61Q 19/00*(2006.01)i; *A61K 8/11*(2006.01)i; *A61K 8/25*(2006.01)i; *A61K 8/73*(2006.01)i; *B01J 13/14*(2006.01)i

FI:    B01J13/14; A61K8/11; A61K8/25; A61K8/73; A61Q19/00; A61Q1/00

According to International Patent Classification (IPC) or to both national classification and IPC

**B.    FIELDS SEARCHED**

Minimum documentation searched (classification system followed by classification symbols)

A61Q1/00; A61Q19/00; A61K8/11; A61K8/25; A61K8/73; B01J13/14

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| X | JP 2011-517323 A (DOW CORNING CORPORATION) 02 June 2011 (2011-06-02)<br>claims 1-12, paragraphs [0001], [0017], [0057], [0063], examples 2-4 | 1-10 |
| X | JP 2019-534139 A (FIRMENICH SA) 28 November 2019 (2019-11-28)<br>claims 1-16, paragraphs [0001], [0023[, [0026], [0067]-[0073], examples 1-11 | 1-10 |
| A | WO 2015/083836 A1 (KAO CORP) 11 June 2015 (2015-06-11)<br>paragraph [0022] | 1-10 |

☐ Further documents are listed in the continuation of Box C.    ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>**12 September 2022** | Date of mailing of the international search report<br>**20 September 2022** |
|---|---|
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

21

INTERNATIONAL SEARCH REPORT
Information on patent family members

International application No.

**PCT/JP2022/026083**

| Patent document cited in search report | | | Publication date (day/month/year) | Patent family member(s) | | | Publication date (day/month/year) |
|---|---|---|---|---|---|---|---|
| JP | 2011-517323 | A | 02 June 2011 | US | 2011/0104221 | A1 | |
| | | | | claims 1-14, paragraphs [0001], [0015], [0063], [0068], examples 2-4 | | | |
| | | | | WO | 2009/106318 | A2 | |
| | | | | EP | 2244689 | A2 | |
| | | | | KR | 10-2010-0117620 | A | |
| | | | | CN | 102014851 | A | |
| JP | 2019-534139 | A | 28 November 2019 | US | 2019/0255502 | A1 | |
| | | | | claims 1-20, paragraphs [0001], [0080]-[0086], examples 1-11 | | | |
| | | | | WO | 2018/054719 | A1 | |
| | | | | EP | 3515587 | A1 | |
| | | | | SG | 11201901972Y | A | |
| | | | | CN | 109715286 | A | |
| | | | | MX | 2019002682 | A | |
| WO | 2015/083836 | A1 | 11 June 2015 | US | 2016/0303531 | A1 | |
| | | | | paragraph [0053] | | | |
| | | | | EP | 3078415 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2862597 A
- WO 2018053356 A
- JP 2012501849 A
- JP 2015128762 A

**Non-patent literature cited in the description**

- **A. LEO.** Comprehensive Medicinal Chemistry. Pergamon Press, 1990, vol. 4, 295